# EUROPEAN PATENT APPLICATION

(11) **EP 3 520 798 A1**
(43) Date of publication of application: **07.08.2019**
(21) Application number: 18154469.3
(22) Date of filing: 31.01.2018
(51) Int. Cl.: A61K 33/10, A61K 9/00, A23L 33/16

(54) **USE OF FUNCTIONALIZED CALCIUM CARBONATE AS ACTIVE INGREDIENT**

(71) Applicant: Omya International AG, 4665 Oftringen (CH)
(72) Inventor: Budde, Tanja, 4805 Brittnau (CH); Sharma, Lalit, 4800 Zofingen (CH)
(74) Representative: Maiwald Patent- und Rechtsanwaltsgesellschaft mbH

(57) **Abstract**

The present invention relates to a dosage form comprising functionalized calcium carbonate serving as active ingredient. The invention further relates to the use of the dosage form as nutritional supplement or as a medicament and to the use of functionalized calcium carbonate as active ingredient, preferably in the field of calcium fortification and in the treatment of calcium deficiency.

## Description

The present invention relates to a dosage form comprising functionalized calcium carbonate serving as active ingredient. The invention further relates to the use of the dosage form as nutritional supplement or as a medicament and to the use of functionalized calcium carbonate as active ingredient, preferably in the field of calcium fortification and in the treatment of calcium deficiency.

Calcium is the most abundant mineral present in the human body. It is continuously utilized by the body and must be replenished by a variety of food sources. The body's use of calcium includes providing rigidity to the skeletal framework, blood clotting, increasing cell membrane permeability, activating a number of enzymes including lipase and adenosine triphosphatase, and acting as a component in the mechanisms of neural transmission and muscular contraction.

Given these representative vital usages of calcium by the body, it is recognized that a dietary calcium deficiency can have adverse effects on an individual's health, which vary in degree depending upon age and sex. It is thought that increased consumption of calcium in early years builds reserves that enable a greater tolerance of a negative calcium balance in later years.

Foods fortified with calcium and calcium supplements are generally considered by some researchers to offer the same net effect as calcium naturally found in food. The most effective order of relative bioavailability or intestinal absorption of various calcium salts is still controversial. There is no consensus among medical authorities as to the effectiveness of one calcium salt over another.

Nevertheless, there are several known factors that affect the absorption of calcium by the human body. In healthy adults approximately 30 % of calcium contained in their diets is absorbed. The absorption of calcium from various foods may range from 10 % to 40 %.

In the art, tricalcium phosphate, calcium lactate, calcium citrate, conventional calcium carbonate and many other calcium compounds have all been used as calcium sources in various calcium fortified products.

Depending on the current standard, the recommended daily intake (RDI) of calcium for teenagers is about 1300 mg/day and ranges from 800 to 1200 mg/day for adults.

Dairy products are rich in calcium and, in some instances, account for as much as 75 % of an individual's dietary intake of calcium. Increased ingestion of dairy products, however, has several drawbacks, which preclude their broad recommendation as a solution for calcium fortification or for use in the treatment of pathological calcium deficiency.

Likewise, calcium citrate is a recognized calcium source and is widely used as food supplement or active ingredient for the purpose of calcium fortification.

Another widely used calcium source is natural or synthetic calcium carbonate, for example ground calcium carbonate obtained from natural sources.

US 2002/0044974 A1 discloses a granular calcium carbonate, wherein the production process comprises the steps of providing an ultrafine calcium carbonate powder having ultrafine particles with an average particle size of no greater than 25 microns, and aggregating said ultrafine powder to form granular calcium carbonate particles, wherein 99 % of which will pass through a 20 mesh screen.

US 6,790,462 B2 discloses a dietary supplement composition, comprising, based on 100 parts by weight of the composition, from about 50 to about 70 parts by weight anhydrous dicalcium phosphate, from about 24 to about 40 parts by weight tricalcium phosphate, and from about 3 to about 11 parts by weight of calcium carbonate.

WO 2007/134158 A2 discloses a pharmaceutical or nutritional composition comprising about 90 % to about 99 % by weight calcium carbonate, about 0.03 % to about 3 % by weight of polyethylene glycol and/or at least one hydrophilic surfactant, and about 1 % to about 10 % by weight of other excipients. The calcium carbonate preferably is a ground natural calcium carbonate.

Calcium carbonate is also frequently used as a food additive to increase the calcium ingestion. Exemplarily, reference is made to US 7,829,127 B2 which is directed to a calcium fortified syrup comprising a syrup comprising sugar and a micronized calcium salt, e.g. micronized calcium carbonate. US 2,166,797 A discloses the use of a precipitated calcium carbonate (i.e. synthetic calcium carbonate) together with a sufficient quantity of phosphates for the fortification of cereals and cereal products. EP 0 195 167 A2 discloses a calcium fortified soy milk which comprises aqueous soy milk, a polyphosphate salt and a human consumable water-soluble calcium salt, such as calcium carbonate.

However, the aforementioned calcium sources have several drawbacks. Drawbacks in case of dairy products include, for example, lactose intolerance, dairy protein allergies, the high levels of cholesterol and cholesterol producing ingredients in dairy products and the high caloric yields of dairy products. Phosphate salts, in particular tricalcium phosphate, have a limited solubility or a low dissolution rate under physiological conditions resulting in a rather limited bioavailability or ingestion of calcium. In case of calcium citrate, the chelating effect of the citrate anion may affect the ingestion of other essential minerals. Flavor defects are also not uncommon. For example, some of the salts used for calcium fortification add taste defects such as tangy taste or even a bitter after-taste.

Therefore, it would be desirable to provide an active ingredient acting as a calcium source or to provide a corresponding dosage form in order to overcome one ore more of the aforementioned drawbacks.

In this respect, one object of the present invention may be seen in the provision of an active ingredient or a corresponding dosage form with an increased calcium release rate. In turn, still another object may be seen in the provision of an active ingredient or a corresponding dosage form for the release of calcium which provides for a higher concentration of dissolved calcium ions in the release environment, e.g. in the mouth, stomach or intestine.

Another object of the present invention thus may be seen in the provision of an active ingredient or a corresponding dosage form for the release of calcium, wherein said active ingredient or dosage form provides for an increased bioavailability of calcium.

Still another object of the present invention may be seen in the provision of an active ingredient or a corresponding dosage form for the release of calcium that allows a less frequent administration, the administration of a smaller amount of active ingredient and a more comfortable administration.

Alone or in combination with the aforementioned objects, another object may be seen in the provision of an active ingredient or a corresponding dosage form for the release of calcium showing one or more of the following characteristics (compared with conventional calcium sources): no or less calories, no or reduced allergies, no or reduced intolerance, and no or less flavour defects.

The foregoing and other problems may be solved by the subject-matter as defined herein in the independent claims.

In this regard, a first aspect of the present invention relates to a dosage form comprising functionalized calcium carbonate, characterized in that the functionalized calcium carbonate serves as active ingredient.

The inventors surprisingly found that the use of functionalized calcium carbonate as active ingredient leads to an increased calcium ion release rate, in particular in an acidic environment. The functionalized calcium carbonate used as the active ingredient is a reaction product of ground natural calcium carbonate (GNCC) or precipitated calcium carbonate (PCC) treated with carbon dioxide and one or more H₃O⁺ ion donors, wherein the carbon dioxide is formed in situ by the H₃O⁺ ion donors treatment and/or is supplied from an external source. In turn, the increased calcium ion release rate is indicative for a higher bioavailability of calcium and a higher efficacy of functionalized calcium carbonate serving as active ingredient for calcium fortification or in the treatment of calcium deficiency.

Another aspect of the present invention relates to the use of the inventive dosage form, comprising functionalized calcium carbonate as active ingredient, as a nutritional supplement.

Still another aspect relates a dosage form according to the present invention for use as a medicament.

Also described herein is a method of treating calcium deficiency, wherein the method comprises a step of administering to a patient a dosage form comprising functionalized calcium carbonate, characterized in that the functionalized calcium carbonate serves as active ingredient.

Still another aspect of the present invention relates to the use of functionalized calcium carbonate as active ingredient, preferably as nutritionally or therapeutically active ingredient.

Still another aspect of the present invention relates to the use of functionalized calcium carbonate as nutrient supplement.

The following terms used in this document shall have the meanings as set forth hereinafter.

The term "functionalized calcium carbonate" as used herein refers to a reaction product of ground natural calcium carbonate (GNCC) or precipitated calcium carbonate (PCC) treated with carbon dioxide and one or more H₃O⁺ ion donors, wherein the carbon dioxide is formed in situ by the H₃O⁺ ion donors treatment and/or is supplied from an external source. Further details in this regard are disclosed hereinbelow.

An "active ingredient" in the meaning of the present document is understood to be a substance that causes or triggers a specific biological activity when applied to a human organism or an animal organism, preferably to a human organism. The term active ingredient as used herein shall include both, active forms and inactive precursors (prodrugs) and is meant as a collective term that shall cover both, nutritionally active ingredients and therapeutically active ingredients. The latter terms are to be understood as a complementary conceptual pair. Accordingly, the term "nutritionally active ingredient" as used herein refers to an active ingredient that is used in a non-therapeutic manner, for example as a supplement for human or animal nutrition, to provide an improvement of performance to an organism being in a normal condition. In turn, the term "therapeutically active ingredient" refers to an active ingredient that is used in a therapeutic manner, for example in human medicines or veterinary medicines, to treat a pathological condition (curative treatment) or that is used to prevent such condition (prophylactic treatment).

A "ground natural calcium carbonate" (GNCC) in the meaning of the present invention is a particulate material obtained from natural calcium carbonate-containing minerals (e.g. chalk, limestone, marble or dolomite) which has been processed in a wet and/or dry comminution step, such as crushing and/or grinding, and optionally has been subjected to further steps such as screening and/or fractionation, for example, by a cyclone or a classifier.

A "precipitated calcium carbonate" (PCC) is a synthesized material, obtained by precipitation following a reaction of carbon dioxide and calcium hydroxide (hydrated lime) in an aqueous environment. Alternatively, precipitated calcium carbonate can also be obtained by reacting calcium- and carbonate salts, for example calcium chloride and sodium carbonate, in an aqueous environment. PCC may have a vateritic, calcitic or aragonitic crystalline form. PCCs are described, for example, in EP 2 447 213 A1, EP 2 524 898 A1, EP 2 371 766 A1, EP 2 840 065 A1, or WO 2013/142473 A1.

Throughout the present document, the term "specific surface area" (in m²/g), which is used to define functionalized calcium carbonate or other materials, refers to the specific surface area as determined by using the BET method (using nitrogen as adsorbing gas).

The "particle size" of all particulate materials disclosed herein, with the exception of ground calcium carbonate and precipitated calcium carbonate, is described as volume-based particle size distribution *dₓ*(vol). Therein, the value *dₓ*(vol) represents the diameter relative to which x % by volume of the particles have diameters less than *dₓ*(vol). This means that, for example, the *d*₂₀(vol) value is the particle size at which 20 vol% of all particles are smaller than that particle size. The *d*₅₀(vol) value is thus the volume median particle size, i.e. 50 vol% of all particles are smaller than that particle size and the *d*₉₈(vol) value, referred to as volume-based top cut, is the particle size at which 98 vol% of all particles are smaller than that particle size.

The "particle size" of ground calcium carbonate and precipitated calcium carbonate herein is described by its distribution of particle sizes *d*ₓ(wt). Therein, the value *d*ₓ(wt) represents the diameter relative to which x % by weight of the particles have diameters less than *d*ₓ(wt). This means that, for example, the *d*₂₀(wt) value is the particle size at which 20 wt% of all particles are smaller than that particle size. The *d*₅₀(wt) value is thus the weight median particle size, i.e. 50 wt% of all particles are smaller than that particle size and the *d*₉₈(wt) value, referred to as weight-based top cut, is the particle size at which 98 wt% of all particles are smaller than that particle size.

For the purpose of the present invention the "porosity" or "pore volume" refers to the intra-particle intruded specific pore volume. The term "pore" is to be understood as describing the space that is found between and/or within particles, i.e. that is formed by the particles as they pack together under nearest neighbour contact (interparticle pores), such as in a powder or a compact, and/or the void space within porous particles (intraparticle pores), and that allows the passage of liquids under pressure when saturated by the liquid and/or supports absorption of surface wetting liquids.

Where an indefinite or definite article is used when referring to a singular noun, e.g. "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

Advantageous embodiments of the inventive dosage form and further aspects are defined in the corresponding dependent claims.

According to one embodiment of the inventive dosage form, the functionalized calcium carbonate serves as nutritionally active ingredient, preferably for the release of calcium.

According to another embodiment, the functionalized calcium carbonate serves as nutritionally active ingredient, preferably for the release of calcium, wherein the inventive dosage form is further characterized in that:
(a) the dosage form further comprises a second nutritionally active ingredient, preferably one or more prebiotics, probiotics, minerals, vitamins, plant extracts, herbal extracts, proteins, enzymes, and/or, polyunsaturated fatty acids such as omega-3 or omega-6 fatty acids, and most preferably one or more minerals such as magnesium, potassium or zinc and/or vitamins such as vitamin D₃ or vitamin K₂; or
(b) the functionalized calcium carbonate is the only nutritionally active ingredient, and most preferably the only active ingredient.

According to still another embodiment, the functionalized calcium carbonate serves as therapeutically active ingredient, preferably for the release of calcium.

According to another embodiment, the functionalized calcium carbonate serves as therapeutically active ingredient, preferably for the release of calcium, wherein the inventive dosage form is further characterized in that:
(a) the dosage form further comprises a second therapeutically active ingredient, preferably one or more prebiotics, probiotics, minerals, vitamins, plant extracts, herbal extracts, proteins, enzymes, and/or, polyunsaturated fatty acids such as omega-3 or omega-6 fatty acids, and most preferably one or more minerals such as magnesium, potassium or zinc and/or vitamins such as vitamin D₃ or vitamin K₂; or
(b) the functionalized calcium carbonate is the only therapeutically active ingredient, and most preferably the only active ingredient.

In another embodiment, the functionalized calcium carbonate is a reaction product of ground natural calcium carbonate (GNCC) or precipitated calcium carbonate (PCC) treated with carbon dioxide and one or more H₃O⁺ ion donors, wherein the carbon dioxide is formed in situ by the H₃O⁺ ion donors treatment and/or is supplied from an external source.

In a preferred embodiment of the present invention, said H₃O⁺ ion donor is selected from strong acids, medium-strong acids, weak acids, acidic salts thereof or mixtures thereof.

In another embodiment, the functionalized calcium carbonate is obtainable by a process comprising the steps of:
(a) providing a suspension of ground natural calcium carbonate (GNCC) or precipitated calcium carbonate (PCC);
(b) adding at least one acid having a pKₐ value of 0 or less at 20 °C or having a pKₐ value from 0 to 2.5 at 20 °C to the suspension of step (a); and
(c) treating the suspension of step (a) with carbon dioxide before, during or after step (b).

In a preferred embodiment, said acid having a pKₐ value of 0 or less at 20 °C is selected from sulphuric acid, hydrochloric acid or mixtures thereof.

In another preferred embodiment, the acid having a pKₐ value from 0 to 2.5 at 20 °C is selected from sulphurous acid, phosphoric acid, oxalic acid or mixtures thereof.

In still another embodiment, the functionalized calcium carbonate is obtainable by a process comprising the steps of:
(a) providing a suspension of ground natural calcium carbonate (GNCC) or precipitated calcium carbonate (PCC);
(b) providing at least one acid;
(c) providing gaseous carbon dioxide; and
(d) contacting the suspension provided in step (a), the at least one acid provided in step (b) and the gaseous carbon dioxide provided in step (c);
wherein
(i) the at least one acid provided in step (b) has a pKₐ of greater than 2.5 and less than or equal to 7 at 20 °C, associated with the ionisation of its first available hydrogen, and a corresponding anion is formed on loss of this first available hydrogen capable of forming a water-soluble calcium salt; and
(ii) following contacting the suspension provided in step (a) and the at least one water-soluble acid provided in step (b), at least one water-soluble salt, which in the case of a hydrogen-containing salt has a pKₐ of greater than 7 at 20 °C, associated with the ionisation of the first available hydrogen, and the salt anion of which is capable of forming water-insoluble calcium salts, is additionally provided.

In a preferred embodiment, said acid added in step (b) is selected from acetic acid, formic acid, propanoic acid or mixtures thereof.

According to still another embodiment of the present invention, the functionalized calcium carbonate has:
(i) a specific surface area of from 10 to 250 m²/g, preferably from 15 to 200 m²/g, more preferably from 20 to 180 m²/g, even more preferably from 25 to 150 m²/g, and most preferably from 35 to 140 m²/g, measured using nitrogen and the BET method according to ISO 9277:2010; and/or
(ii) a volume-based particle size *d*₅₀(vol) of from 0.8 to 75 µm, preferably from 1 to 50 µm, more preferably 2 to 40 µm, even more preferably from 2.5 to 30 µm, and most preferably from 3 to 15 µm; and/or
(iii) a volume-based particle size *d*₉₈(vol) of from 2 to 150 µm, preferably from 5 to 100 µm, more preferably 8 to 50 µm, even more preferably from 10 to 35 µm, and most preferably from 12 to 25 µm; and/or
(iv) an intra-particle intruded specific pore volume in the range from 0.1 to 2.3 cm³/g, more preferably from 0.2 to 2.0 cm³/g, even more preferably from 0.4 to 1.8 cm³/g and most preferably from 0.6 to 1.6 cm³/g, calculated from mercury porosimetry measurement.

According to still another embodiment, the dosage form is an oral dosage form, preferably a solid oral dosage form, and most preferably the dosage form is a tablet, a capsule, a chewable tablet, a lozenge, an orodispersible tablet, a powder, a granulate or a an effervescent tablet.

According to still another embodiment, the dosage form further comprises one or more formulation aids, preferably one or more formulation aids selected from fillers, binders, disintegrants, diluents, lubricants, film forming agents, adhesives, buffers, adsorbents, natural or synthetic scenting agents, natural or synthetic flavouring agents, natural or synthetic coloring agents, natural or synthetic sweeteners, natural or synthetic odor masking agents, natural or synthetic flavour masking agents, natural or synthetic taste masking agents, and natural and/or synthetic mouthfeel enhancers.

In still another embodiment, the inventive dosage form is used as a nutritional supplement for calcium fortification.

In still another embodiment, the inventive dosage form is a dosage form for use in the treatment of calcium deficiency.

According to another embodiment of the present invention, the functionalized calcium carbonate is used as nutritionally active ingredient, preferably for the release of calcium.

According to still another embodiment, the functionalized calcium carbonate is used as therapeutically active ingredient, preferably for the release of calcium.

In still another embodiment, the functionalized calcium carbonate serves as nutrient supplement for calcium fortification.

In the following, details and preferred embodiments of the inventive dosage form will be disclosed. It is to be understood that these details and embodiments also apply to the use of said dosage form as nutritional supplement, to said dosage form for use as a medicament, to the use of functionalized calcium carbonate as active ingredient as well as to the use of functionalized calcium carbonate as nutrient supplement.

### (A) Functionalized calcium carbonate

The active ingredient of the present invention is a functionalized calcium carbonate (FCC).

It is appreciated that the functionalized calcium carbonate can be one or a mixture of different kinds of functionalized calcium carbonate(s). In one embodiment of the present invention, the functionalized calcium carbonate comprises, preferably consists of, one kind of functionalized calcium carbonate. Alternatively, the functionalized calcium carbonate comprises, preferably consists of, two or more kinds of functionalized calcium carbonates. For example, the functionalized calcium carbonate comprises, preferably consists of, two or three kinds of functionalized calcium carbonates. Preferably, the functionalized calcium carbonate comprises, more preferably consists of, one kind of functionalized calcium carbonate.

The functionalized calcium carbonate is a reaction product of ground natural calcium carbonate (GNCC) or precipitated calcium carbonate (PCC) treated with carbon dioxide and one or more H₃O⁺ ion donors, wherein the carbon dioxide is formed in situ by the H₃O⁺ ion donors treatment and/or is supplied from an external source. Because of the reaction of ground natural calcium carbonate or precipitated calcium carbonate with carbon dioxide and the one or more H₃O⁺ ion donors, functionalized calcium carbonate may comprise GNCC or PCC and at least one water-insoluble calcium salt other than calcium carbonate.

In a preferred embodiment, said functionalized calcium carbonate comprises GNCC or PCC and at least one water-insoluble calcium salt other than calcium carbonate which is present on at least part of the surface of said GNCC or PCC.

An H₃O⁺ ion donor in the context of the present invention is a Brønsted acid and/or an acid salt.

In a preferred embodiment of the invention, the functionalized calcium carbonate is obtained by a process comprising the steps of:
(a) providing a suspension of ground natural calcium carbonate (GNCC) or precipitated calcium carbonate (PCC);
(b) adding at least one acid having a pKₐ value of 0 or less at 20 °C, or having a pKₐ value from 0 to 2.5 at 20 °C to the suspension provided in step (a); and
(c) treating the suspension provided in step (a) with carbon dioxide before, during or after step (b).

According to another embodiment, the functionalized calcium carbonate is obtained by a process comprising the steps of:
(a) providing a ground natural calcium carbonate (GNCC) or precipitated calcium carbonate (PCC);
(b) providing at least one water-soluble acid;
(c) providing gaseous carbon dioxide; and
(d) contacting said GNCC or PCC provided in step (a), the at least one acid provided in step (b) and the gaseous carbon dioxide provided in step (c);
characterized in that
(i) the at least one acid provided in step (b) has a pKₐ of greater than 2.5 and less than or equal to 7 at 20 °C, associated with the ionisation of its first available hydrogen, and a corresponding anion is formed on loss of this first available hydrogen capable of forming a water-soluble calcium salt; and
(ii) following contacting the at least one water-soluble acid provided in step (b) and the GNCC or PCC provided in step (a), at least one water-soluble salt, which in the case of a hydrogen-containing salt has a pKₐ of greater than 7 at 20 °C, associated with the ionisation of the first available hydrogen, and the salt anion of which is capable of forming water-insoluble calcium salts, is additionally provided.

The source of calcium carbonate, e.g., ground natural calcium carbonate (GNCC), preferably is selected from calcium carbonate-containing minerals selected from the group comprising marble, chalk, limestone and mixtures thereof. Natural calcium carbonate may comprise further naturally occurring components such as magnesium carbonate, alumino silicate etc. According to one embodiment, natural calcium carbonate, such as GNCC, comprises aragonitic, vateritic or calcitic mineralogical crystal forms of calcium carbonate or mixtures thereof.

In general, the grinding of ground natural calcium carbonate may be performed in a dry or wet grinding process and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulverizer, a shredder, a de-clumper, a knife cutter, or other such equipment known to the skilled person. In case the ground natural calcium carbonate comprises wet ground calcium carbonate, the grinding step may be performed under conditions such that autogenous grinding takes place and/or by horizontal ball milling, and/or other such processes known to the skilled person. The wet processed ground natural calcium carbonate thus obtained may be washed and dewatered by well-known processes, e.g., by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

As already indicated hereinabove, a precipitated calcium carbonate (PCC) in the meaning of the present invention is a synthesized material, generally obtained by precipitation following a reaction of carbon dioxide and calcium hydroxide in an aqueous environment or by precipitation of calcium and carbonate ions, for example CaCl₂ and Na₂CO₃, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained aqueous PCC slurry can be mechanically dewatered and dried.

According to one embodiment of the present invention, the precipitated calcium carbonate comprises aragonitic, vateritic or calcitic mineralogical crystal forms of calcium carbonate or mixtures thereof.

Precipitated calcium carbonate may be ground prior to the treatment with carbon dioxide and at least one H₃O⁺ ion donor by the same means as used for grinding natural calcium carbonate and described above.

According to one embodiment of the present invention, the natural or precipitated calcium carbonate is in form of particles having a weight median particle size *d*₅₀(wt) of from 0.05 to 10.0 µm, preferably from 0.2 to 5.0 µm, more preferably from 0.4 to 3.0 µm, most preferably from 0.6 to 1.2 µm, and especially 0.7 µm According to a further embodiment of the present invention, the natural or precipitated calcium carbonate is in form of particles having a top cut particle size *d*₉₈(wt) of from 0.15 to 55 µm, preferably from 1 to 40 µm, more preferably from 2 to 25 µm, most preferably from 3 to 15 µm, and especially 4 µm.

The natural or precipitated calcium carbonate may be used dry or suspended in water. Preferably, a corresponding aqueous slurry has a content of natural or precipitated calcium carbonate within the range of from 1 to 90 wt%, more preferably from 3 to 60 wt%, even more preferably from 5 to 40 wt%, and most preferably from 10 to 25 wt%, based on the total weight of said slurry.

The one or more H₃O⁺ ion donor used for the preparation of functionalized calcium carbonate may be any strong acid, medium-strong acid, or weak acid, or mixtures thereof, generating H₃O⁺ ions under the preparation conditions. According to the present invention, the at least one H₃O⁺ ion donor can also be an acid salt, generating H₃O⁺ ions under the preparation conditions.

According to one embodiment, the at least one H₃O⁺ ion donor is a strong acid having a pKₐ of 0 or less at 20 °C.

According to another embodiment, the at least one H₃O⁺ ion donor is a medium-strong acid having a pKₐ value from 0 to 2.5 at 20 °C. If the pKₐ at 20 °C is 0 or less, the acid is preferably selected from sulphuric acid, hydrochloric acid, or mixtures thereof. If the pKₐ at 20 °C is from 0 to 2.5, the H₃O⁺ ion donor is preferably selected from H₂SO₃, H₃PO₄, oxalic acid, or mixtures thereof. The at least one H₃O⁺ ion donor can also be an acid salt, for example, HSO₄⁻ or H₂PO₄⁻, being at least partially neutralized by a corresponding cation such as Li⁺, Na⁺ or K⁺, or HPO₄²⁻, being at least partially neutralized by a corresponding cation such as Li⁺, Na⁺, K⁺, Mg²⁺ or Ca²⁺. The at least one H₃O⁺ ion donor can also be a mixture of one or more acids and one or more acid salts.

According to still another embodiment, the at least one H₃O⁺ ion donor is a weak acid having a pKₐ value of greater than 2.5 and less than or equal to 7, when measured at 20 °C, associated with the ionisation of the first available hydrogen, and having a corresponding anion, which is capable of forming water-soluble calcium salts. Subsequently, at least one water-soluble salt, which in the case of a hydrogen-containing salt has a pKₐ of greater than 7, when measured at 20 °C, associated with the ionisation of the first available hydrogen, and the salt anion of which is capable of forming water-insoluble calcium salts, is additionally provided. According to a more preferred embodiment, the weak acid has a pKₐ value from greater than 2.5 to 5 at 20 °C, and more preferably the weak acid is selected from the group consisting of acetic acid, formic acid, propanoic acid and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of dropwise addition, this addition preferably takes place within a time period of 10 min. It is more preferred to add said salt in one step.

According to one embodiment of the present invention, the at least one H₃O⁺ ion donor is selected from the group consisting of hydrochloric acid, sulphuric acid, sulphurous acid, phosphoric acid, citric acid, oxalic acid, acetic acid, formic acid and mixtures thereof. Preferably the at least one H₃O⁺ ion donor is selected from the group consisting of hydrochloric acid, sulphuric acid, sulphurous acid, phosphoric acid, oxalic acid, H₂PO₄⁻, being at least partially neutralized by a corresponding cation such as Li⁺, Na⁺ or K⁺, HPO₄²⁻, being at least partially neutralized by a corresponding cation such as Li⁺, Na⁺, K⁺, Mg²⁺ or Ca²⁺ and mixtures thereof, more preferably the at least one acid is selected from the group consisting of hydrochloric acid, sulphuric acid, sulphurous acid, phosphoric acid, oxalic acid, or mixtures thereof. A particularly preferred H₃O⁺ ion donor is phosphoric acid.

The one or more H₃O⁺ ion donor can be added to the suspension as a concentrated solution or a more diluted solution. Preferably, the molar ratio of the H₃O⁺ ion donor to the natural or precipitated calcium carbonate is from 0.01:1 to 4:1, more preferably from 0.02:1 to 2:1, even more preferably from 0.05:1 to 1:1 and most preferably from 0.1:1 to 0.58:1.

In another preferred embodiment, the at least one H₃O⁺ ion donor is selected from the group consisting of hydrochloric acid, sulphuric acid, sulphurous acid, phosphoric acid, citric acid, oxalic acid, acetic acid, formic acid and mixtures thereof, wherein the molar ratio of the H₃O⁺ ion donor to the natural or precipitated calcium carbonate is from 0.01:1 to 4:1, more preferably from 0.02:1 to 2:1, even more preferably from 0.05:1 to 1:1 and most preferably from 0.1:1 to 0.58:1.

In a particularly preferred embodiment, the at least one H₃O⁺ ion donor is a mixture of phosphoric acid and citric acid, more preferably the molar ratio of the H₃O⁺ ion donor to the natural or precipitated calcium carbonate is from 0.01:1 to 4:1, more preferably from 0.02:1 to 2:1, even more preferably from 0.05:1 to 1:1 and most preferably from 0.1:1 to 0.58:1. In this embodiment, phosphoric acid is preferably used in excess relative to citric acid.

As an alternative, it is also possible to add the H₃O⁺ ion donor to the water before the natural or precipitated calcium carbonate is suspended.

In a next step, the natural or precipitated calcium carbonate is treated with carbon dioxide. If a strong acid such as sulphuric acid or hydrochloric acid is used for the H₃O⁺ ion donor treatment of the natural or precipitated calcium carbonate, the carbon dioxide is automatically formed. Alternatively or additionally, the carbon dioxide can be supplied from an external source.

H₃O⁺ ion donor treatment and treatment with carbon dioxide can be carried out simultaneously which is the case when a strong or medium-strong acid is used. It is also possible to carry out H₃O⁺ ion donor treatment first, e.g., with a medium strong acid having a pKₐ in the range of 0 to 2.5 at 20 °C, wherein carbon dioxide is formed in situ, and thus, the carbon dioxide treatment will automatically be carried out simultaneously with the H₃O⁺ ion donor treatment, followed by the additional treatment with carbon dioxide supplied from an external source.

Preferably, the concentration of gaseous carbon dioxide in the suspension is, in terms of volume, such that the ratio (volume of suspension):(volume of gaseous carbon dioxide) is from 1:0.05 to 1:20, even more preferably 1:0.05 to 1:5.

In a preferred embodiment, the H₃O⁺ ion donor treatment step and/or the carbon dioxide treatment step are repeated at least once, more preferably several times. According to one embodiment, the at least one H₃O⁺ ion donor is added over a time period of at least about 5 min, preferably at least about 10 min, typically from about 10 to about 20 min, more preferably about 30 min, even more preferably about 45 min, and sometimes about 1 h or more.

Subsequent to the H₃O⁺ ion donor treatment and carbon dioxide treatment, the pH of the aqueous suspension, measured at 20 °C, naturally reaches a value of greater than 6.0, preferably greater than 6.5, more preferably greater than 7.0, even more preferably greater than 7.5, thereby preparing the functionalized natural or precipitated calcium carbonate as an aqueous suspension having a pH of greater than 6.0, preferably greater than 6.5, more preferably greater than 7.0, even more preferably greater than 7.5.

Further details about the preparation of the functionalized natural calcium carbonate are disclosed in WO 00/39222 A1, WO 2004/083316 A1, WO 2005/121257 A2, WO 2009/074492 A1, EP 2 264 108 A1, EP 2 264 109 A1 and US 2004/0020410 A1, the content of these references herewith being included in the present document.

Similarly, functionalized precipitated calcium carbonate may be obtained. As can be taken in detail from WO 2009/074492 A1, functionalized precipitated calcium carbonate is obtained by contacting precipitated calcium carbonate with H₃O⁺ ions and with anions being solubilized in an aqueous medium and being capable of forming water-insoluble calcium salts, in an aqueous medium to form a slurry of functionalized precipitated calcium carbonate, wherein said functionalized precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by H₃O⁺ ions, where said H₃O⁺ ions are provided solely in the form of a counter ion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said H₃O⁺ ions may be provided by the addition of an acid or an acid salt of said anion, or the addition of an acid or an acid salt which simultaneously serves to provide all or part of said excess solubilized calcium ions.

In a further preferred embodiment of the preparation of the functionalized natural or precipitated calcium carbonate, the natural or precipitated calcium carbonate is reacted with the acid and/or the carbon dioxide in the presence of at least one compound selected from the group consisting of silicate, silica, aluminium hydroxide, earth alkali aluminate such as sodium or potassium aluminate, magnesium oxide, aluminium sulphate or mixtures thereof. Preferably, the at least one silicate is selected from an aluminium silicate, a calcium silicate, or an earth alkali metal silicate.

In another preferred embodiment, said at least one compound is aluminium sulphate hexadecahydrate. In a particularly preferred embodiment, said at least one compound is aluminium sulphate hexadecahydrate, wherein the at least one H₃O⁺ ion donor is selected from the group consisting of hydrochloric acid, sulphuric acid, sulphurous acid, phosphoric acid, citric acid, oxalic acid, acetic acid, formic acid and mixtures thereof, more preferably the molar ratio of said H₃O⁺ ion donor to the natural or precipitated calcium carbonate is from 0.01:1 to 4:1, more preferably from 0.02:1 to 2:1, even more preferably from 0.05:1 to 1:1 and most preferably from 0.1:1 to 0.58:1.

The foregoing components can be added to an aqueous suspension comprising the natural or precipitated calcium carbonate before adding the acid and/or carbon dioxide.

Alternatively, the foregoing components can be added to the aqueous suspension of natural or precipitated calcium carbonate while the reaction of natural or precipitated calcium carbonate with an acid and carbon dioxide has already started. Further details about the preparation of the functionalized natural or precipitated calcium carbonate in the presence of at least one silicate and/or silica and/or aluminium hydroxide and/or earth alkali aluminate component(s) are disclosed in WO 2004/083316 A1, the content of this reference herewith being included in the present document.

The functionalized calcium carbonate can be kept in suspension, optionally further stabilized by a dispersant. Conventional food grade dispersants known to the skilled person can be used.

Alternatively, the aqueous suspension described above can be dried, thereby obtaining the solid (i.e. dry or containing as little water that it is not in a fluid form) functionalized natural or precipitated calcium carbonate in the form of granules or a powder.

The functionalized calcium carbonate may have different particle shapes, such as e.g., the shape of roses, golf balls and/or brains.

In a preferred embodiment, the functionalized calcium carbonate has a specific surface area of from 10 to 250 m²/g, preferably from 15 to 200 m²/g, more preferably from 20 to 180 m²/g, even more preferably from 25 to 150 m²/g, and most preferably from 35 to 140 m²/g, measured using nitrogen and the BET method according to ISO 9277:2010. In a further embodiment, the functionalized calcium carbonate has a specific surface area of 120 m²/g or less, more preferably from 60 to 120 m²/g, and most preferably from 70 to 105 m²/g, measured using nitrogen and the BET method according to ISO 9277:2010. For example, the functionalized calcium carbonate may have a specific surface area of from 75 to 100 m²/g, measured using nitrogen and the BET method according to ISO 9277:2010.

It may furthermore be preferred that the functionalized calcium carbonate particles have a volume median grain diameter *d*₅₀(vol) of from 0.8 to 75 µm, preferably from 1 to 50 µm, more preferably from 2 to 40 µm, even more preferably from 2.5 to 30 µm, and most preferably from 3 to 15 µm. According to another preferred embodiment, the functionalized calcium carbonate particles have a volume median grain diameter *d*₅₀(vol) of from 1.5 to 12 µm, preferably from 2 to 5 µm or from 6 to 10 µm.

It may furthermore be preferred that the functionalized calcium carbonate particles have a grain diameter *d*₉₈(vol) of from 2 to 150 µm, preferably from 5 to 100 µm, more preferably from 8 to 50 µm, even more preferably from 10 to 35 µm, and most preferably from 12 to 25 µm. According to another preferred embodiment, the functionalized calcium carbonate particles have a volume median grain diameter *d*₉₈(vol) of from 5 to 20 µm, preferably from 8 to 12 µm or from 13 to 18 µm

According to another embodiment, the functionalized calcium carbonate has an intra-particle intruded specific pore volume in the range from 0.1 to 2.3 cm³/g, more preferably from 0.2 to 2.0 cm³/g, especially preferably from 0.4 to 1.8 cm³/g and most preferably from 0.6 to 1.6 cm³/g, calculated from mercury porosimetry measurement.

The intra-particle pore size of the functionalized calcium carbonate preferably is in a range of from 0.004 to 1.6 µm, more preferably in a range of between 0.005 to 1.3 µm, especially preferably from 0.006 to 1.15 µm and most preferably of 0.007 to 1.0 µm, e.g., 0.004 to 0.50 µm determined by mercury porosimetry measurement.

### (B) The dosage form

The inventive dosage form is a dosage form comprising functionalized calcium carbonate, wherein the functionalized calcium carbonate serves as active ingredient, preferably for the release of calcium.

As already described hereinabove, the functionalized calcium carbonate can serve as nutritionally active ingredient or therapeutically active ingredient, which depends on the purpose of administration. A nutritionally active ingredient in the meaning of the present invention is used in a non-therapeutic manner to provide an improvement of performance to an organism being in a normal condition, whereas a therapeutically active ingredient is used in a therapeutic manner, i.e. for the purpose of curative or prophylactic treatment.

It has been found, surprisingly, that the use of functionalized calcium carbonate as active ingredient leads to an increased calcium ion release rate, in particular in an acidic environment. In turn, the increased calcium ion release rate is indicative for a higher bioavailability of calcium and a higher efficacy of functionalized calcium carbonate serving as active ingredient for calcium fortification or in the treatment of calcium deficiency. This is surprising because the inventors believe that the acidic treatment of GNCC or PCC leads to the formation of water-insoluble calcium salts (other than calcium carbonate) on at least part of the surface of said GNCC or PCC.

Apart from functionalized calcium carbonate, which serves as active ingredient, the dosage form according to the present invention can comprise further active ingredients, including both, nutritionally active ingredients and therapeutically active ingredients.

Therefore, in one embodiment, the dosage form comprises a further nutritionally active ingredient and/or a further therapeutically active ingredient, independently from whether the functionalized calcium carbonate serves as nutritionally or therapeutically active ingredient. Depending on the purpose of administration, the further nutritionally or therapeutically active ingredient can be selected from one or more prebiotics, probiotics, minerals, vitamins, plant extracts, herbal extracts, proteins, enzymes, and/or polyunsaturated fatty acids such as omega-3 or omega-6 fatty acids, and most preferably one or more minerals such as magnesium, potassium or zinc and/or vitamins such as vitamin D₃ or vitamin K₂.

In still another embodiment of the inventive dosage form, the functionalized calcium carbonate is the only active ingredient contained in the dosage form, meaning that the dosage form contains no further active ingredients apart from functionalized calcium carbonate.

In a further specific embodiment, the functionalized calcium carbonate serves as nutritionally active ingredient, wherein the functionalized calcium carbonate is the only nutritionally active ingredient, preferably the only active ingredient, contained in the dosage form.

In a further specific embodiment, the functionalized calcium carbonate serves as therapeutically active ingredient, wherein the functionalized calcium carbonate is the only therapeutically active ingredient, preferably the only active ingredient, contained in the dosage form.

The inventive dosage form can be administered by any conceivable way. However, in one embodiment, the dosage form is an oral dosage form, preferably a solid oral dosage form, and most preferably the dosage form is a tablet, a capsule, a chewable tablet, a lozenge, an orodispersible tablet, a powder, a granulate or a an effervescent tablet.

In any of the aforementioned dosage forms, the inventive dosage form can comprise one or more formulation aids. Preferably, the formulation aids are selected from fillers, binders, disintegrants, diluents, lubricants, film forming agents, adhesives, buffers, adsorbents, natural or synthetic scenting agents, natural or synthetic flavouring agents, natural or synthetic coloring agents, natural or synthetic sweeteners, natural or synthetic odor masking agents, natural or synthetic flavour masking agents, natural or synthetic taste masking agents, and natural and/or synthetic mouthfeel enhancers.

Functionalized calcium carbonate is a particulate mineral-containing material and may be also administered as is. This particularly applies in cases where the functionalized calcium carbonate has a volume-based particle size *d*₅₀(vol) of from 0.8 to 75 µm, preferably from 1 to 50 µm, more preferably 2 to 40 µm, even more preferably from 2.5 to 30 µm, and most preferably from 3 to 15 µm and/or a volume-based particle size *d*₉₈(vol) of from 2 to 150 µm, preferably from 5 to 100 µm, more preferably 8 to 50 µm, even more preferably from 10 to 35 µm, and most preferably from 12 to 25 µm. Therefore, in another embodiment, the inventive dosage form consists of functionalized calcium carbonate, wherein the functionalized calcium carbonate serves as active ingredient, preferably for the release of calcium. Such dosage forms consisting of functionalized calcium carbonate, wherein the functionalized calcium carbonate serves as active ingredient, include solid oral dosage forms, preferably tablets, powders and granules.

### (C) Further aspects

Another aspect disclosed herein relates to the use of the dosage form according to the present invention as a nutritional supplement, preferably for calcium fortification. As explained hereinabove, an active ingredient in the meaning of the present document causes or triggers a specific biological activity when applied to a human organism or an animal organism, preferably to a human organism. Therefore, the term "nutritional supplement" as used herein shall refer to both, food supplements (i.e. for human nutrition) as well as feed supplements (i.e. for animal nutrition). In one embodiment, the use as food supplement is preferred.

More specifically, one aspect of the present invention relates to the use of a dosage form comprising functionalized calcium carbonate as a nutritional supplement, preferably for calcium fortification, characterized in that the functionalized calcium carbonate serves as active ingredient, preferably the functionalized calcium carbonate serves as nutritionally active ingredient, more preferably for the release of calcium.

In a preferred embodiment according to the previous aspect, the dosage form comprises a further nutritionally active ingredient, preferably one or more prebiotics, probiotics, minerals, vitamins, plant extracts, herbal extracts, proteins, enzymes, and/or polyunsaturated fatty acids such as omega-3 or omega-6 fatty acids, and most preferably one or more minerals such as magnesium, potassium or zinc and/or vitamins such as vitamin D₃ or vitamin K₂; or the functionalized calcium carbonate is the only active ingredient.

Still another aspect disclosed herein relates to a dosage form according to the present invention for use as a medicament, preferably for use in the treatment of calcium deficiency. Considering that an active ingredient in the meaning of the present document causes or triggers a specific biological activity when applied to a human organism or an animal organism, the term "medicament" as used herein refers to both, human medicines and veterinary medicines. In a preferred embodiment, the medicament is a human medicine.

More specifically, one aspect of the present invention relates to a dosage form comprising functionalized calcium carbonate for use as a medicament, preferably for use in the treatment of calcium deficiency, characterized in that the functionalized calcium carbonate serves as active ingredient, preferably the functionalized calcium carbonate serves as therapeutically active ingredient, more preferably for the release of calcium.

In a preferred embodiment according to the previous aspect, the dosage form comprises a further therapeutically active ingredient, preferably one or more prebiotics, probiotics, minerals, vitamins, plant extracts, herbal extracts, proteins, enzymes, and/or polyunsaturated fatty acids such as omega-3 or omega-6 fatty acids, and most preferably one or more minerals such as magnesium, potassium or zinc and/or vitamins such as vitamin D₃ or vitamin K₂; or the functionalized calcium carbonate is the only active ingredient.

The skilled person will appreciate that, where appropriate, any embodiments and details disclosed in connection with the inventive dosage form, e.g. those relating to the functionalized calcium carbonate and the different administration forms, will apply analogously to the use of said dosage form.

Still another aspect of the present invention relates to the use of functionalized calcium carbonate as active ingredient, preferably as nutritionally or therapeutically active ingredient.

In a preferred embodiment according to the previous aspect, the functionalized calcium carbonate is used as nutritionally active ingredient or as therapeutically active ingredient, preferably for the release of calcium.

Again, the skilled person will appreciate that, where appropriate, any embodiments and details disclosed in connection with the inventive dosage form and its uses, in particular those relating to the functionalized calcium carbonate, will apply analogously to the use of use said functionalized calcium carbonate as an active ingredient.

While it is possible to administer the dosage form comprising functionalized calcium carbonate directly, for example as an oral dosage form serving as a nutritional supplement, it is also possible to use the functionalized calcium carbonate as such as a nutrient supplement, independently from whether it would be suitable to be administered directly, i.e. as a dosage form. In general, functionalized calcium carbonate can be used as a nutrient supplement in both, food products and animal feed.

Therefore, one further aspect of the present invention relates to the use of functionalized calcium carbonate as nutrient supplement, preferably as nutrient supplement in food products or in animal feed, more preferably in food products. Preferred food products are infant formula, instant beverages, sports food, confectionary products such as candies, chewing gums and chocolates, baked goods such as cakes, muffins, soft and hard baked cookies and breads, cereals, cereal bars, dairy products, and non-dairy milk products such as soy milk, almond milk, rice milk, oat milk or derived products. Preferred animal feed includes compressed or pelleted animal feed.

Also disclosed herein is a method of treating calcium deficiency, wherein the method comprises a step of administering to a patient a dosage form comprising functionalized calcium carbonate, characterized in that the functionalized calcium carbonate serves as active ingredient.

In one embodiment according to the previous aspect, the functionalized calcium carbonate serves as therapeutically active ingredient, preferably for the release of calcium.

In still another embodiment according to the previous aspect, the dosage form is an oral dosage form, preferably a solid oral dosage form, and most preferably the dosage form is a tablet, a capsule, a chewable tablet, a lozenge, an orodispersible tablet, a powder, a granulate or a an effervescent tablet.

In still another embodiment, the dosage form further comprises a therapeutically active ingredient, preferably one or more prebiotics, probiotics, minerals, vitamins, plant extracts, herbal extracts, proteins, enzymes, and/or polyunsaturated fatty acids such as omega-3 or omega-6 fatty acids, and most preferably one or more minerals such as magnesium, potassium or zinc and/or vitamins such as vitamin D3 or vitamin K2; or the functionalized calcium carbonate is the only active ingredient.

### EXAMPLES

The scope and interest of the invention may be better understood on basis of the following examples which are intended to illustrate embodiments of the present invention.

### (A) Analytical methods

All parameters defined throughout the present document and mentioned in the following examples are based on the following measuring methods:

### Specific surface area (SSA)

The specific surface area (in m²/g) is determined using the BET method (using nitrogen as adsorbing gas), which is well known to the skilled man (ISO 9277:2010). The total surface area (in m²) of the filler material is then obtained by multiplication of the specific surface area and the mass (in g) of the corresponding sample.

### Particle size distribution

All particle sizes described herein, with the exception of the particle sizes of the ground calcium carbonate that was used for the production of the functionalized calcium carbonate and that of precipitated calcium carbonate, refer to the volume-based particle size distribution *dₓ*(vol). The volume-based median particle size *d*₅₀(vol) and top cut *d*₉₈(vol) are evaluated using a Malvern Mastersizer 3000 Laser Diffraction System (Malvern Instruments Plc., Great Britain). The raw data obtained by the measurement is analyzed using the Fraunhofer theory. The methods and instruments are known to the skilled person and are commonly used to determine particle size distributions. Measurements were carried out on the dry products.

The particle size of the ground calcium carbonate that was used for the production of the functionalized calcium carbonate is described herein as weight-based particle size distribution *dₓ*(wt). The same applies to precipitated calcium carbonate. The weight-based median particle size *d*₅₀(wt) and top cut *d*₉₈(wt) are measured by the sedimentation method, which is an analysis of sedimentation behaviour in a gravimetric field. The measurement is made with a Sedigraph™ 5120 of Micromeritics Instrument Corporation, USA. The method and the instrument are known to the skilled person and are commonly used to determine particle size distributions. The measurement is carried out in an aqueous solution of 0.1 wt% Na₄P₂O₇. The samples are dispersed using a high speed stirrer and sonication.

### Specific pore volume

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 µm. The equilibration time used at each pressure step is 20 s. The sample material is sealed in a 3 cm³ chamber powder penetrometer for analysis. The data are corrected for mercury compression, penetrometer expansion and sample material elastic compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 1996, 35(5), 1753 - 1764).

The total pore volume seen in the cumulative intrusion data is separated into two regions with the intrusion data from 214 µm down to about 1 to 4 µm showing the coarse packing of the sample between any agglomerate structures contributing strongly. Below these diameters lies the fine interparticle packing of the particles themselves. If they also have intraparticle pores, then this region appears bimodal, and by taking the specific pore volume intruded by mercury into pores finer than the modal turning point, i.e. finer than the bimodal point of inflection, we thus define the specific intraparticle pore volume. The sum of these three regions gives the total overall pore volume of the powder, but depends strongly on the original sample compaction/settling of the powder at the coarse pore end of the distribution.

By taking the first derivative of the cumulative intrusion curve, the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

### (B) Examples

The following examples are not to be construed to limit the scope of the claims in any manner whatsoever.

### Preparation of functionalized calcium carbonates

### Example 1A - FCC 1

FCC 1 has a *d*₅₀ = 4.44 µm, a *d*₉₈ = 11.0 µm, a SSA = 54.7 m²g⁻¹ and an intra-particle intruded specific pore volume of 0.807 cm³/g (for the pore diameter range of 0.004 to 0.47 µm).

FCC 1 was obtained by preparing 350 litres of an aqueous suspension of ground calcium carbonate in a mixing vessel by adjusting the solids content of a ground limestone calcium carbonate from Omya SAS, Orgon having a weight-based median particle size of 1.3 µm, as determined by sedimentation, such that a solids content of 10 wt%, based on the total weight of the aqueous suspension, is obtained.

Whilst mixing the slurry at a speed of 6.2 m/s, 11.2 kg phosphoric acid was added in form of an aqueous solution containing 30 wt% phosphoric acid to said suspension over a period of 20 minutes at a temperature of 70 °C. After the addition of the acid, the slurry was stirred for additional 5 minutes, before removing it from the vessel and drying using a jet-dryer.

### Example 1B - FCC 2

FCC 2 has a *d*₅₀ = 5.58 µm, *d*₉₈ = 15.0 µm, a SSA = 90.6 m²/g and an intra-particle intruded specific pore volume of 1.71 cm³/g (for the pore diameter range of 0.004 to 0.47 µm).

FCC 2 was obtained by preparing 1500 liters of an aqueous suspension of ground calcium carbonate in a mixing vessel by adjusting the solids content of a ground limestone calcium carbonate from Omya SAS, Orgon, having a weight-based median particle size of 0.6µm, as determined by sedimentation, such that a solids content of 10.0 wt%, based on the total weight of the aqueous suspension, is obtained.

Whilst mixing the slurry rapidly, 80 kg phosphoric acid was added in form of an aqueous solution containing 20 wt% phosphoric acid to said suspension over a period of 60 minutes at a temperature of 62 °C. After the addition of the acid, the slurry was stirred for additional 5 minutes, before removing it from the vessel and drying using a jet-dryer.

### Calcium release testings

The functionalized calcium carbonates prepared according to the above protocols were tested in terms of their calcium release rate:

| **Materials*** | ***d*₅₀ [µm]** | ***d*₉₈ [µm]** | **SSA [cm²/g]** | **Pore volume [cm³/g]** |
|---|---|---|---|---|
| **FCC 1** | 4.44 | 11.0 | 54.7 | 0.807 |
| **FCC 2** | 5.58 | 15.0 | 90.6 | 1.711 |

The following materials were used for comparative purposes:

| **Materials*** | ***d*₅₀ [µm]** | ***d*₉₈ [µm]** | **SSA [cm²/g]** | **Pore volume [cm³/g]** |
|---|---|---|---|---|
| **Calcium citrate tetrahydrate** | 6.83 | 80 | | |
| **TCP 1 (nano)** | 4.57 | 33 | 52.9 | 0.519 |
| **TCP 2 (non-nano)** | 4 | 30 | 7.6 | 0.157 |
| **NCC 1** | 1.83 | 9 | 4.1 | |

| | | | | |
|---|---|---|---|---|
| **FCC* = *functionalized calcium carbonate* *TCP* **=** *tricalcium phosphate* *NCC = natural calcium carbonate* | | | | |

### Example 2 - Calcium ion potential in solution

In this example, the calcium ion concentration at pH 3 was analysed to investigate the release of calcium ions in an acidic environment.

One litre of distilled water was provided in a beaker and adjusted to pH 3 by addition of 1 M HCl (Sigma-Aldrich) under continuous stirring to obtain an acidified medium.

For each calcium ion source (FCC 1, TCP 1 and NCC 1) 80 mL of the acidified medium was used. To these 80 mL of acidified medium, the calcium ion source FCC 1, TCP 1 or NCC 1 was added in an amount to provide a calcium ion concentration that is equal to 20 mg/L.

The release of calcium ions over time was investigated by measuring the electrical potential using a calcium-selective ion probe (Mettler-Toledo DX240) and a reference electrode (Mettler-Toledo DX200). The voltage developed across the membrane is directly linked to the amount of calcium ions in the solution. Before every measurement, the electrodes were cleaned with distilled water. In addition, the calcium-selective ion probe (Mettler-Toledo DX240) was dried with a tissue.

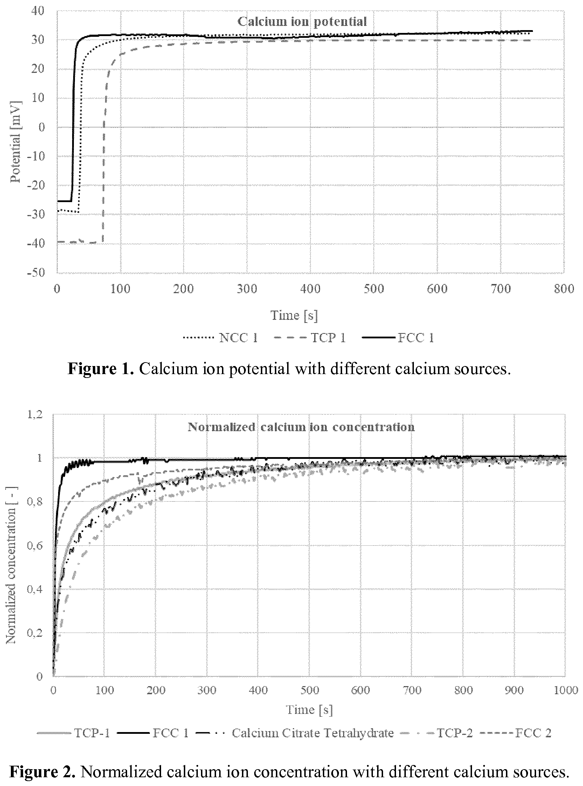

The measurements are illustrated in Figure 1. It was shown that the calcium ion release rate is higher in case of functionalized calcium carbonate (FCC 1) compared to conventional calcium carbonate (NCC 1) and nano-scale tricalcium phosphate (TCP 1).

### Example 3 - Normalized calcium ion concentration in solution

In these trials, an ionic strength adjuster was used in addition in order to keep the ionic strength at a constant level and to exclude any impact of variable ionic strengths on the measured calcium ion activity.

Ionic strength adjuster solution: 53.49 g of NH₄Cl were dissolved in a 1 L volumetric flask with distilled water.

Reaction solution: 14.6 g of 25% HCl were diluted in a 1 L volumetric flask with distilled water. 10 mL of this solution and 100 mL of the ionic strength adjuster solution were added and then diluted in a 1 L volumetric flask with distilled water.

Calcium standard solution: 5 mL of a 1000 ppm calcium standard for ion-selective electrodes (ISE) were added in a 100 mL volumetric flask and diluted with distilled water.

Calibration solutions: 10 mL of ionic strength adjuster were added in 5 volumetric flasks and a calculated volume of calcium standard solution was added to each one.

Measurement: The release of calcium ions over time was investigated by measuring the electrical potential. All solutions (calibration solutions and sample solutions) were measured by using a calcium-selective ion probe (Mettler-Toledo DX240) and a reference electrode (Mettler-Toledo DX200). Before every measurement, the electrodes were cleaned with distilled water. In addition, the calcium-selective ion probe (Mettler-Toledo DX240) was dried with a tissue. All samples were stirred at the same stirring rate with a magnetic stirrer. A calibration curve was measured before every sample series. Potentials were converted into concentrations based on the calibration measurement.

Calibration measurement: Calibration solutions were measured in 100 mL memo beakers and stirred with a magnetic stirrer. The potential had to be constant before it was noted.

Sample measurement: Each 1 L of reaction solution as described above were provided in a 1 L beaker and the calcium ion source (FCC 1, FCC 2, TCP 1, TCP 2, calcium citrate tetrahydrate) was added in an amount to provide a calcium ion concentration that is equal to 20 mg/L after the measured potential of the reaction solution reached a constant level. Measurements were stopped after 20 min.

The measurements are illustrated in Figure 2. It was shown that the calcium ion release rate is higher in case of functionalized calcium carbonate (FCC 1 and FCC 2) compared to conventional calcium sources (TCP 1, TCP 2, calcium citrate tetrahydrate). The concentrations were normalized for each sample based on the final concentration of calcium ions so that a normalized concentration of 1 means that the final concentration level is reached.

## Claims

1. A dosage form comprising functionalized calcium carbonate, **characterized in that** the functionalized calcium carbonate serves as active ingredient.

2. The dosage form according to claim 1, **characterized in that** the functionalized calcium carbonate serves as nutritionally active ingredient, preferably for the release of calcium.

3. The dosage form according to claim 2, **characterized in that**:
(a) the dosage form further comprises a second nutritionally active ingredient, preferably one or more prebiotics, probiotics, minerals, vitamins, plant extracts, herbal extracts, proteins, enzymes, and/or, polyunsaturated fatty acids such as omega-3 or omega-6 fatty acids, and most preferably one or more minerals such as magnesium, potassium or zinc and/or vitamins such as vitamin D₃ or vitamin K₂; or
(b) the functionalized calcium carbonate is the only nutritionally active ingredient, and most preferably the only active ingredient.

4. The dosage form according to claim 1, **characterized in that** the functionalized calcium carbonate serves as therapeutically active ingredient, preferably for the release of calcium.

5. The dosage form according to claim 4, **characterized in that**:
(a) the dosage form further comprises a second therapeutically active ingredient, preferably one or more prebiotics, probiotics, minerals, vitamins, plant extracts, herbal extracts, proteins, enzymes, and/or, polyunsaturated fatty acids such as omega-3 or omega-6 fatty acids, and most preferably one or more minerals such as magnesium, potassium or zinc and/or vitamins such as vitamin D₃ or vitamin K₂; or
(b) the functionalized calcium carbonate is the only therapeutically active ingredient, and most preferably the only active ingredient.

6. The dosage form according to any of claims 1 to 5, **characterized in that** the functionalized calcium carbonate is a reaction product of ground natural calcium carbonate (GNCC) or precipitated calcium carbonate (PCC) treated with carbon dioxide and one or more H₃O⁺ ion donors, wherein the carbon dioxide is formed in situ by the H₃O⁺ ion donors treatment and/or is supplied from an external source.

7. The dosage form according to claim 6, **characterized in that** the H₃O⁺ ion donor is selected from strong acids, medium-strong acids, weak acids, acidic salts thereof or mixtures thereof.

8. The dosage form according to any of claims 1 to 5, **characterized in that** the functionalized calcium carbonate is obtainable by a process comprising the steps of:
(8a) providing a suspension of ground natural calcium carbonate (GNCC) or precipitated calcium carbonate (PCC);
(8b) adding at least one acid having a pKₐ value of 0 or less at 20 °C or having a pKₐ value from 0 to 2.5 at 20 °C to the suspension of step (8a); and
(8c) treating the suspension of step (8a) with carbon dioxide before, during or after step (8b).

9. The dosage form according to claim 8, **characterized in that** the acid having a pKₐ value of 0 or less at 20 °C is selected from sulphuric acid, hydrochloric acid or mixtures thereof.

10. The dosage form according to claim 8, **characterized in that** the acid having a pKₐ value from 0 to 2.5 at 20 °C is selected from sulphurous acid, phosphoric acid, oxalic acid or mixtures thereof.

11. The dosage form according to any of claims 1 to 5, **characterized in that** the functionalized calcium carbonate is obtainable by a process comprising the steps of:
(11a) providing a suspension of ground natural calcium carbonate (GNCC) or precipitated calcium carbonate (PCC);
(11b) providing at least one acid;
(11c) providing gaseous carbon dioxide; and
(11d) contacting the suspension provided in step (11a), the at least one acid provided in step (11b) and the gaseous carbon dioxide provided in step (11c);
wherein
(i) the at least one acid provided in step (11b) has a pKₐ of greater than 2.5 and less than or equal to 7 at 20 °C, associated with the ionisation of its first available hydrogen, and a corresponding anion is formed on loss of this first available hydrogen capable of forming a water-soluble calcium salt; and
(ii) following contacting the suspension provided in step (11a) and the at least one water-soluble acid provided in step (11b), at least one water-soluble salt, which in the case of a hydrogen-containing salt has a pKₐ of greater than 7 at 20 °C, associated with the ionisation of the first available hydrogen, and the salt anion of which is capable of forming water-insoluble calcium salts, is additionally provided.

12. The dosage form according to claim 11, **characterized in that** the acid added in step (11b) is selected from acetic acid, formic acid, propanoic acid or mixtures thereof.

13. The dosage form according to any of claims 1 to 12, **characterized in that** the functionalized calcium carbonate has:
(i) a specific surface area of from 10 to 250 m²/g, preferably from 15 to 200 m²/g, more preferably from 20 to 180 m²/g, even more preferably from 25 to 150 m²/g, and most preferably from 35 to 140 m²/g, measured using nitrogen and the BET method according to ISO 9277:2010; and/or
(ii) a volume-based particle size *d*₅₀(vol) of from 0.8 to 75 µm, preferably from 1 to 50 µm, more preferably 2 to 40 µm, even more preferably from 2.5 to 30 µm, and most preferably from 3 to 15 µm; and/or
(iii) a volume-based particle size *d*₉₈(vol) of from 2 to 150 µm, preferably from 5 to 100 µm, more preferably 8 to 50 µm, even more preferably from 10 to 35 µm, and most preferably from 12 to 25 µm; and/or
(iv) an intra-particle intruded specific pore volume in the range from 0.1 to 2.3 cm³/g, more preferably from 0.2 to 2.0 cm³/g, even more preferably from 0.4 to 1.8 cm³/g and most preferably from 0.6 to 1.6 cm³/g, calculated from mercury porosimetry measurement.

14. The dosage form according to any of claims 1 to 13, **characterized in that** the dosage form is an oral dosage form, preferably a solid oral dosage form, and most preferably the dosage form is a tablet, a capsule, a chewable tablet, a lozenge, an orodispersible tablet, a powder, a granulate or a an effervescent tablet.

15. The dosage form according to any of claims 1 to 14, **characterized in that** the dosage form further comprises one or more formulation aids, preferably one or more formulation aids selected from fillers, binders, disintegrants, diluents, lubricants, film forming agents, adhesives, buffers, adsorbents, natural or synthetic scenting agents, natural or synthetic flavouring agents, natural or synthetic coloring agents, natural or synthetic sweeteners, natural or synthetic odor masking agents, natural or synthetic flavour masking agents, natural or synthetic taste masking agents, and natural and/or synthetic mouthfeel enhancers.

16. Use of a dosage form as defined in any of claims 1, 2, 3, 6, 7, 8, 9, 10, 11, 12, 13, 14 or 15 as a nutritional supplement.

17. The use according to claim 16, **characterized in that** the dosage form is used as a nutritional supplement for calcium fortification.

18. A dosage form as defined in any of claims 1, 4, 5, 6, 7, 8, 9, 10, 11, 12, 13, 14 or 15 for use as a medicament.

19. The dosage form according to claim 18, **characterized in that** the dosage form is a dosage form for use in the treatment of calcium deficiency.

20. Use of functionalized calcium carbonate as active ingredient, preferably as nutritionally or therapeutically active ingredient.

21. The use according to claim 20, **characterized in that** the functionalized calcium carbonate is used as nutritionally active ingredient, preferably for the release of calcium.

22. The use according to claim 20, **characterized in that** the functionalized calcium carbonate is used as therapeutically active ingredient, preferably for the release of calcium.

23. Use of functionalized calcium carbonate as nutrient supplement.

24. The use according to claim 23, **characterized in that** the functionalized calcium carbonate serves as nutrient supplement for calcium fortification.
